# EUROPEAN PATENT APPLICATION

(11) **EP 4 710 911 A2**
(43) Date of publication of application: **18.03.2026**
(21) Application number: 26155982.7
(22) Date of filing: 19.05.2021
(51) Int. Cl.: A61H 23/00

(54) **METHODS AND APPARATUS TO MANAGE MEIBOMIAN GLAND DEFICIENCIES**

(30) Priority: 20.05.2020 AU 2020901615; 20.05.2020 AU 2020901616; 20.05.2020 AU 2020901617
(62) Divisional of application: 21809582.6
(71) Applicant: Nthalmic Holding Pty Ltd, Sydney NSW 2019 (AU); Zhong Jing Wei Shi (Suzhou) Optical Technology Ltd., Suzhou City, Jiangsu Province (CN)
(72) Inventor: BAKARAJU, Ravi Chandra, Sydney NSW, 2019 (AU); EHRMANN, Klaus, Sydney NSW, 2019 (AU)
(74) Representative: Becker, Eberhard

(57) **Abstract**

A plurality of methods and apparatus for managing deficiencies of the meibomian gland (102, 103) lipid production and delivery, including wearable fabrics configured with a matrix of micro- and nano- electromechanical materials, or composite fibres of yarn that are substantially made from carbon nanotubes, graphene, spider silk, natural silk, denim, cotton, metals, or combination thereof. Other methods and apparatus include infrared (109a, 109b) and ultrasound (109c) energy sources within a kit aimed at personalised therapies for improving overall health of the eyelids, while providing for adjunctive relief for associated dry eye symptoms. The management kit may comprise a wearable fabric or spectacle frame configured with infrared emitters (109a) and sensors (109b), a handheld imaging device (605) and software containing dosage and administration information coupled with information on improving eyelid hygiene which is reviewed and periodically updated using user-specific information.

## Description

### FIELD OF THE INVENTION

The disclosure relates to methods and apparatus for managing deficiencies of the meibomian gland lipid production and delivery utilising and/or applying thermal and mechanical energy to the ocular adnexa.

### BACKGROUND

Meibomian gland dysfunction is one of the most common causes of dry eye disorders. The meibomian glands are located within the inner tarsal plate of the eyelids. Functionally, they are sebaceous glands responsible for the supply of essential oils, referred to as meibum, which facilitate lubrication on the surface of the eye. There are approximately 30 to 40 glands located on the upper eyelids and 15 to 25 on the lower eyelids. They spread the essential oil over the surface of the eye to prevent the evaporation of the tear film.

Dysfunction of these meibomian glands causes the ocular tears to evaporate more rapidly, which may eventually lead to the symptoms of dry eyes, burning sensation, and/or irritation of eyes. The two common underlying reasons of dysfunction of the meibomian glands are either the solidification of the oil in the glands or obstruction of the pores which otherwise would allow the flow of oil onto the surface of the eye.

The recommended clinical management regime involves warm compresses and lid massage up to four times per day for 15-minute episodes, and adjunctive use of lubricants to ameliorate dry eye related symptoms.

### DISCUSSION OF RELATED ART AND NEED FOR THIS DISCLOSURE

The US patent application US2019/015401, incorporated in its entirety, refers to a method of using infrared and ultrasound energy to treat meibomian gland dysfunction. It utilises invasive pressure plates and handheld devices which require a medical practitioner to administer the procedure.

The LipiFlow thermal pulsation system described in US patent 9314369B2, incorporated in its entirety, discloses another in-office medical device that applies sufficient heat to the eyelids to melt waxy deposits in the meibomian glands. At the same time, it applies pulsed pressure to the eyelid to open and thoroughly express the contents of the glands. While the treatment efficacy with a comprehensive device is trusted, the shortcoming of the procedure is its invasive approach and the need for the procedure to be performed under strict supervision of a medical practitioner. Further, the instrument costs for an eye-care practitioner and ongoing out-of-pocket costs for the end-user can be prohibitively expensive.

There is clearly a need for development of a method and apparatus to offer a device that is non-invasive and allows self-administration of the procedure from the comfort of the home.

Also, a treatment that can be administered in both open and closed eye conditions is preferred.

### SUMMARY OF THE INVENTION

Carbon has various crystalline structures, of which, carbon nanotube (1-D material) and graphene (2-D material), have been previously contemplated for micro and nano electromechanical systems. The carbon nanotubes are nanometre sized, tube shaped, molecules rolled into a tube.

A carbon nanotube can be single-walled or multi-walled, which is dependent upon the methods of manufacturing. The diameters of carbon nanotubes are in the order of tens of nanometres to hundreds of nanometres. Their longitudinal hollow structures impart unique mechanical, electrical, and chemical properties to the materials. These carbon nanotubes typically are electrically conductive and mechanically strong and stiff along their length. The carbon nanotubes typically also have a relatively high aspect ratio, that is length to diameter ratio. These carbon nanotubes can be spun into fibre yarns by mechanically fastening the twisted aggregates of individual tubes together.

Graphene has a higher thermal conductivity than carbon nanotubes, and its electrical resistance is lower than that of copper or silver. In some embodiments, the 2D sheets of graphene may be preferable over the 1D carbon nanotubes. Spider silks and silkworm silks comprise of proteinaceous fibres which are substantially composed of non-essential amino acids. The composition of the non-essential amino acids present in the silk, defines their physical properties, for example, elasticity, tensile strength, thermal and electrical conductivity, or resistance. Due to these robust electro-mechanical properties, the use of carbon nanotube, or graphene sheets, or spider silk, or silkworm silk, or combinations thereof, as reinforcements in fabrics configured with composite materials for both structural and functional applications would be advantageous, as disclosed herein for the management of meibomian gland deficiencies. Other advantages of the current disclosure, such as, replacing the need for relatively large, expensive, cumbersome and energy inefficient systems with newer alternatives that are user-friendly, energy efficient with precise heating elements, making it relatively inexpensive may eliminate the need for a substantial aid from the medical practitioner to conduct the procedure and manage the meibomian gland dysfunction and other dry eye conditions will become evident in this disclosure.

Certain embodiments of this disclosure are directed towards a substantially non-invasive method and/or device for the management of meibomian gland deficiencies that use, at least in part, thermal energy to warm up the eyelids, and thereby increasing the temperature of the meibomian glands through principles of thermal conduction. In one specific embodiment of the present disclosure, the contemplated wearable device may be a fabric placed in juxtaposition of the eyelids of the user.

Certain embodiments of the disclosure are configured by selecting a specific arrangement of the nanofibres that offer thermal radiation that is most efficiently absorbed by the lipids or the tissue surrounding the glands, wherein a warming effect can be achieved without substantially overheating other tissues around the ocular adnexa. In certain embodiments, an integrated temperature sensor may be weaved into the wearable fabric providing feedback to maintain the preferred temperature range of heat application on the eyelids.

In certain embodiments, the wearable fabric may also be configured with a plurality of miniaturised ultrasound emitters that may be positioned in proximity to the eyelid margins to either function in a continuous or intermit mode or at a pre-programmed mode or via user triggered intervals for liquefaction of the calcified or keratinised orifices of the eyelids of the wearer.

In certain embodiments, the wearable fabric may also be further configured with compact artificial muscles capable of applying contractile strokes mimicking the effects of lid massaging. For example, use of coiled fibres may be attractive candidates for such purposes, owing to their simple construction and the possibility of actuating via thermal, electrical, or chemical stimulation. In one particular embodiment, the carbon fibres may be coupled with polydimethylsiloxane compound to produce the artificial muscle actions and contractile strokes through the fibre. In one implementation of the present disclosure, the wearable fabric may be configured such that the user may be able to keep their eyes open for any routine visual tasks that they would otherwise take part on a normal day; this would obviously require a different fabric configuration that would result in much smaller coverage area compared to the closed eye condition. In another embodiment, the wearable fabric may be further configured with a micromotor or a piezo-activator.

In another implementation of the present disclosure, the wearable fabric may be configured such that the user may only be able to use the wearable device in closed eye conditions. In such embodiments that are designed specifically for closed eye application, it may be preferable to integrate a sensor that detects closed eyelids, and heating energy is only applied while the eyelids are closed for prolonged periods. This may provide the user full control over the treatment and also increases efficiency as more eyelid tissue is exposed to the treatment while the lids are closed. Other variations to apply the treatment overnight on the closed eyes may be contemplated by a person skilled in the art. In certain embodiments of the present disclosure, a fabric configured with a matrix of electro-mechanical nano materials or nano fibres that are substantially made from nano-scaled carbon nanotubes, or graphene plates, or graphene sheets, or spider silk fibres, or natural silk fibres, or other metal fibres, and the bundles of such fibres are further configured to be substantially aligned along at least one specified direction or axis of the eyelids of the wearer or user of the device.

In some embodiments, the micromotor or piezo-activator may be configured to provide a vibration in the tangential direction of the eyelids of the wearer; wherein the vibrations of the at least one micromotor or piezo-activator generates a pressure wave from the origin to the orifice of the meibomian glands with a pressure range of 0.005 to 0.5 N/mm² and a frequency range from 0.5 to 3 Hz.

In some embodiments, the apparatus may comprise of multiple layers of nanofibres substantially made of carbon nanotubes or graphene plates or sheets which may be further configured to suit the shape of the eyelids of an individual wearer. A plurality of electro-mechanical configurations of the nanofibres at a plurality of locations on the eyelids are contemplated in the current disclosure. The alignment of the nanofibres of the contemplated wearable device may further facilitate heating and mechanical vibrations in at least one specific direction or axis of the eyelids of the wearer or user of the device. The disclosure generally relates to methods and apparatus for managing deficiencies of the meibomian gland lipid production and delivery, using thermal/ infrared energy and/or lid-massaging through mechanical vibrations facilitated using a wearable fabric substantially made of stretchable conductive composite yarn, for example, nanofibres which are made of graphene, or carbon nano tubes, or silkworm silk, or spider silk, or metal nanowires or combinations thereof. Other suitable yarns may also be contemplated.

One embodiment of the current disclosure relates to the field of wearable fabrics made of nanofibres and composite materials. In particular, the wearable fabric is configured with a specific and controlled degree of preferred orientation or arrangement of the bundles of nanofibres in proximity or in contact with the eyelids of the wearer. The disclosure further relates to a specific type of wearable fabrics that are configured with yarns comprising bundles of nanofibres that exhibit desired elastic modulus, tensile strength, thermal conductivity, and/or electrical conductivity or resistance.

Certain other embodiments of the disclosure relate to a comprehensive management kit for meibomian gland deficiencies that comprises convenient combinations and personalised management solutions for improving overall health of the eyelids, ocular surface, or ocular adnexa, and provide adjunctive relief for associated dry eye symptoms for a wearer.

For example, one embodiment discloses a management kit comprising: (a) a spectacle device to be used in open-eye condition; (b) a wearable fabric to be used in closed eye condition; (c) a handheld imaging device for monitoring the health of the glands; and (d) a mobile application.

For example, the spectacle device of the management kit may be configured with a plurality of infrared emitters of a certain wavelength to provide thermal energy and a plurality of infrared sensors to read the surface temperature of the eyelids. For example, the wearable fabric of the management kit may consist of a substantial number of fibre bundles made of composite yarn, which are further configured with carbon nanotubes, graphene, spider silk, or natural silk. For example, the handheld imaging device of the management kit may include multi-frequency self-focussing micro mechanical ultrasound transducer and receiver for monitoring the health of the glands.

In certain embodiments, the management kit further comprises software containing dosage and administration information coupled with information on improving eyelid hygiene, which may be constantly reviewed and updated using periodic information obtained from the user profile. The current disclosure is related to a kit for managing deficiencies of the meibomian gland lipid production and delivery of a wearer. The present disclosure generally relates to a personalised kit for maintaining eyelid hygiene and healthy ocular surface and ameliorate any associated symptoms of dry eye for a wearer.

The disclosure generally relates to a kit, methods, and apparatus for managing deficiencies of the meibomian gland lipid production and delivery, the kit comprising of a spectacle frame, a wearable fabric, and a portable imaging system. The spectacle frame and wearable fabric are further configured with micro or nano electromechanical constituents that utilise infrared/thermal energy, lid-massaging through mechanical vibrations facilitated using a spectacle frame or wearable fabric to improve the flow of oil from the meibomian glands of the wearer.

Certain embodiments of this disclosure are directed towards a substantially non-invasive method and/or device for the management of meibomian gland deficiencies that uses, at least in part, infrared light energy to increase the surface temperature of the eyelids, and thereby increasing the temperature of the meibomian glands through principles of thermal conduction. In one specific embodiment of the present disclosure, the contemplated ophthalmic device may be a spectacle or a wearable frame, while in another contemplated embodiment example of the present disclosure, the ophthalmic device may be an eye mask or goggles, or similar wearable configured in the proximity of the eyelids or ocular adnexa.

Certain spectacle or wearable embodiments of the disclosure are configured by selecting a wavelength that is most efficiently absorbed by the lipids or the tissue surrounding the glands, wherein a warming effect can be achieved without substantially overheating other tissues around the ocular adnexa. An integrated, non-contact, temperature sensor mounted on the spectacle or wearable embodiment provides feedback to maintain the preferred temperature range. Several other modes of operation are envisaged.

A spectacle or wearable frame can be fitted with rows of infrared light emitting diodes (LEDs) that illuminate the upper or lower eyelids and lid margins either continuously or intermittently at pre-programmed or user-triggered intervals. In one implementation, it may be preferable to integrate a sensor that detects closed eyelids, and thermal energy is only applied while the eyelids are closed for prolonged periods. This provides the user full control over the treatment and also increases efficiency as more eyelid tissue is exposed to the treatment while the lids are closed.

Another variation of the contemplated disclosure may include an apparatus that facilitates application of the device overnight on the closed eyes to improve efficacy of the management of meibomian gland dysfunction. In such an embodiment, the user would be required to wear an infrared emitting eye mask over the eyes to receive extended periods of heat treatment through the array of infrared energy sources configured into the wearable fabric mask device. As the wearable fabric would be in contact with the eyelids, additional forms of treatment could be integrated. For example, this may include application of mechanical vibration or massaging, ultrasonic cavitation of blocked orifices or photo-acoustic imaging to manage the condition.

Certain embodiments disclose the use of an array of individual infrared LEDs, wherein the use of the array of individual infrared sources, a pattern of selectively illuminated areas may be created. Such an arrangement of the embodiment may be advantageous for stimulating the increase in lipid generation and flow. Certain embodiments of the contemplated disclosure using the infrared light energy sources over a range of wavelengths of around 1.2 to 1.7 µm aimed to maximise the management, as these wavelength regions are known to be absorbed by the lipids most efficiently.

The disclosure relates generally to methods and apparatus for treating dry eye including infrared light and/or ultrasound energy. One of the embodiments of the disclosure relates to an ophthalmic device that uses infrared light energy to manage deficiencies of the meibomian gland lipid production and delivery. There is a need for further development of a method and apparatus to offer a device that is non-invasive and/or that may allow administration of the procedure from the comfort of the home of an end-user or without necessitating the aid of medical professional.

Another embodiment of the current disclosure generally relates to the field of wearable fabrics made of nanofibres and composite materials. In particular, the wearable fabrics configured with a specific and controlled degree of preferred orientation or arrangement of the bundles of nanofibres along the eyelids of the wearer. The disclosure generally relates to type of wearable fabrics that are configured with yarns comprising bundles of nanofibres that exhibit desired elastic modulus, tensile strength, thermal conductivity, and/or electrical conductivity.

Certain embodiments of this disclosure are directed to a method and/or apparatus for the management of meibomian gland deficiencies via the use of infrared light to increase the temperature of the eyelids and/or the ocular adnexa and thereby stimulating the flow of oil from the meibomian glands through thermal conduction.

Certain other embodiments of the present disclosure contemplate selection of wavelengths that are most efficiently absorbed by the lipids or the tissue surrounding the glands and/or ocular adnexa, such that the resultant warming effect may be achieved without overheating other tissues around the ocular adnexa.

In certain embodiments, an integrated, non-contact temperature sensor is configured to provide feedback to maintain the preferred temperature range for an individual end-user.

Throughout the disclosure, several other modes of operation are envisaged. In one specific embodiment, the ophthalmic device may be a spectacle or a wearable frame, while in another embodiment example, the ophthalmic device may be a wearable fabric or similar wearable configured in the proximity of the eyelids.

### BRIEF DESCRIPTION OF THE FIGURES

Figure 1 illustrates the side view of an open eye, the ocular surface comprising the black (pupil) and white parts of the eye (conjunctiva), the iris, the eyelids and meibomian glands located in the eyelids, respectively. Figure 1 further illustrates a frontal view structural diagram of the wearable fabrics designed for the eyelids configured with nano fibres arranged in the vertical and the horizontal directions, which may be used to manage an eye with meibomian gland dysfunction, according to certain aspects of the present disclosure.
Figure 2 illustrates a structural diagram showing wearable fabrics on the eyelids with a mesh-type alignment of the nanofibres which may be used to manage an eye with meibomian gland dysfunction according to certain aspects of the present disclosure. The duration of wear is approximately between 5 and 20 minutes per session and the device is powered using external rechargeable batteries.
Figure 3 illustrates a structural diagram showing the posterior view of a spectacle or a wearable device which may be used to manage an eye with meibomian gland dysfunction according to certain aspects of the present disclosure.
Figure 4 illustrates a structural diagram showing the entire spectacle or wearable device which may be used to manage an eye with meibomian gland dysfunction according to certain aspects of the present disclosure.
Figure 5 illustrates the top view of a kit for managing meibomian gland deficiencies, including a wearable spectacle frame, a wearable fabric, a handheld ultrasound imager, an USB data transmitter, as disclosed herein.
Figure 6 showcases the functioning of a handheld ultrasound imaging system that relays the measured signals onto a handheld device (for example a mobile phone or a tablet).
Figure 7 illustrates a flow chart of a method that may be used to manage an eye with meibomian gland deficiency according to certain aspects of the present disclosure, as disclosed herein.
Figure 8 illustrates a flow chart of a method directed by personalised data of the user that may be used to manage an eye with meibomian gland deficiency according to certain aspects of the present disclosure, as disclosed herein.

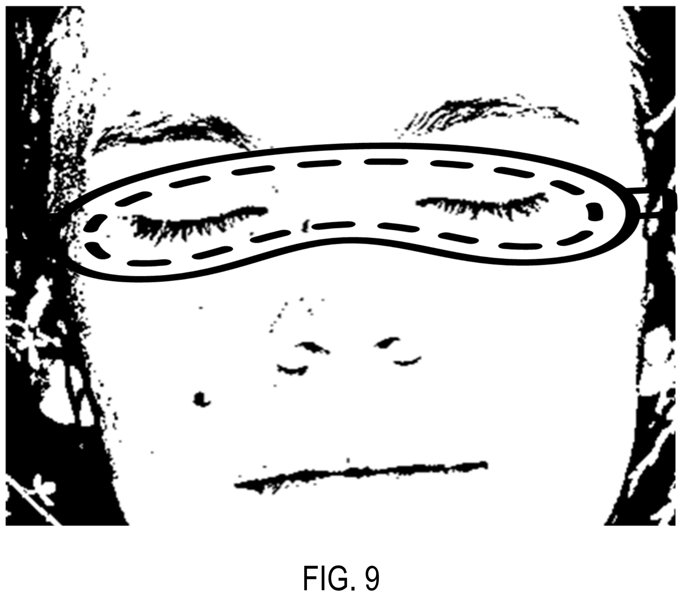

Figure 9 illustrates a frontal view of one of the embodiments of the present disclosure worn by a wearer as a mask.

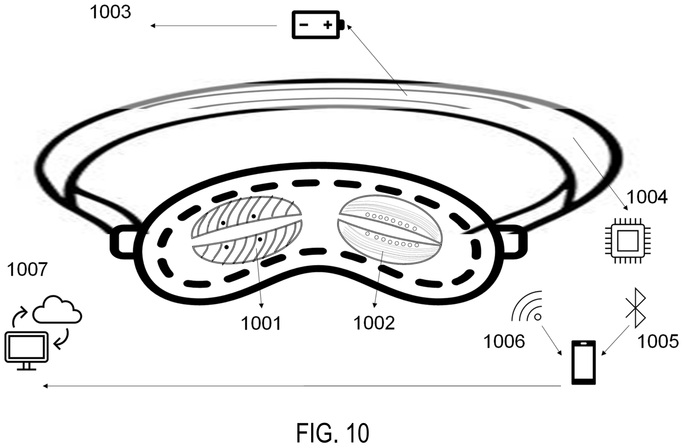

Figure 10 illustrates individual components of one of the embodiments described in Figure 9 of the present disclosure worn.

### DETAILED DESCRIPTION

The following description is provided in relation to several embodiments that may share common characteristics of the disclosure. It is to be understood that one or more structural characteristics of one embodiment may be combined with one or more functional characteristics of other embodiments. The relevant subject headings should not be used in construing the scope of the claims or the claim limitations.

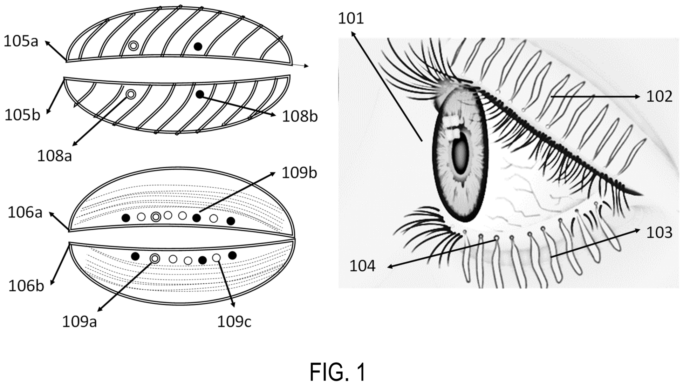

The right-half of Figure 1 showcases the side view of the ocular surface, highlighting the anterior part of the eye (101, cornea, iris and pupil), the eyelids and the meibomian glands of the upper (102) and lower (103) eyelids; and the orifices/openings of the eyelids (104).

The left-half (top) of Figure 1 showcases the front view of a wearable fabric of the current disclosure, which is configured with an array of nanofibres (105a & 105b, line striations), arranged in the direction of the meibomian glands (vertical). In this example, the array of nanofibres of the wearable fabric, at least in part, comprises stretchable conductive composite yarn materials made of carbon nanotubes and graphene sheets.

In another variation of the disclosed embodiment, the array of nanofibres of the wearable fabric, at least in part, may comprise, natural, spider or artificial silk. In another variation of the disclosed embodiment, the array of nanofibres of the wearable fabric, may comprise at least in part, a metal or an alloy of metals. In another variation of the disclosed embodiment, the array of nanofibres of the wearable fabric, may comprise at least in part, cotton or denim, and an elastic formed of a polymer or copolymer of a polyamide, a polyester, a polyolefin, and mixtures thereof.

The wearable fabric may be configured with an IR emitter (108a) configured in proximity to the upper and/or lower eyelids for provision of additional heating of the eyelids. The wearable fabric is configured with temperature sensors or detectors (108b) configured in proximity to the upper and/or lower eyelids. The temperature sensors or detectors may be used to obtain feedback on the surface temperature from the eyelids. Further, the electronic circuit of the wearable fabric may be powered by an externally placed or internally placed battery, for example a 12V button cell. Further this battery may be configured such that it can be recharged via a docking station.

In addition to the infrared emitters, infrared sensors, ultrasound transducers, the wearable fabric may be further configured with a micromotor or piezo-activator to provide a vibration in the tangential direction of the eyelids of the wearer; wherein the vibrations of the at least one micromotor or piezo-activator generates a pressure wave from the origin to the orifice of the meibomian glands with a pressure range of 0.005 to 0.5 N/mm² and a frequency range from 0.5 to 3 Hz.

The left-half (bottom) of Figure 1 also showcases the side view of another contemplated wearable fabric which is configured with an array of nanofibres (106a & 106b, line striations), arranged in the direction perpendicular to the meibomian glands (horizontal), infrared emitters (109a) and infrared sensors (109b) configured in proximity of the eyelid margin.

Further, the wearable fabric of Figure 1 is also configured with ultrasound emitters (109c) to facilitate fragmentation of calcified orifices of the eyelids which in turn would aid in increase of meibum through the orifices. The ultra-sound emitters are configured on the eyelid margins. The electronic circuit of the wearable fabric may be powered by an externally placed or internally placed battery, for example a lithium-ion rechargeable flexible battery.

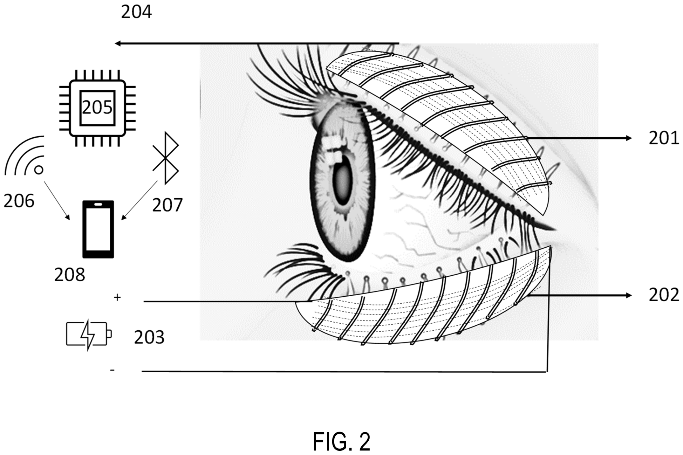

Figure 2 showcases the side view of another contemplated wearable fabric which is configured with an array of nanofibres (201 & 202, crossline striations), arranged in both the directions parallel and perpendicular to the meibomian glands (horizontal and vertical). Further, the wires adjoining the positive and negative terminals may be operated by an externally placed or internally placed battery (203). In some embodiments, the nanofibres may be doped with other elements, for example boron. Various other configurations of Figures 1 and 2 can be contemplated to reduce the disclosure to practice. A device may be designed to make it simple and inexpensive, on the other hand, more complex embodiments with similar functionalities may also be contemplated. For example, the power source may either be configured as an extension of the wearable fabric, on the other hand, the power may be sourced externally through a cable. In another example, the power source may be housed in a headband alleviating the load within the proximity of the eyelids, orbit, or the face.

Further, the temperature control could be via a manual switch, or alternatively an electronic chip configured onto the wearable fabric may provide feedback based on the readings obtained through the infrared sensors depicted in Figure 2. Various other configurations may also be contemplated. To obtain more accurate temperature readings, it may be advantageous to disable the nanofibres briefly before and while the temperature is measured.

The wearable fabric of Figures 1 and 2 may be further configured with ultrasound emitters close to the lower and upper margins of the eyelids. An onboard rechargeable battery sources the emitters, sensors, and the micro-chip computer may be contemplated. The data or information processed on the micro-computer (205) may be transmitted to a smart device 208 (mobile phone or tablet) via use of standard Bluetooth connection (207) or wireless (206) functionality. The transmitted data or the information may be related to one of the following, for example, the voltage settings of the wearable fabric, the current setting of the wearable fabric, the resistance setting of the nanofibres, the frequency setting of the ultrasound emitter, the power setting of the ultrasound emitter, the temperature reading from the infrared sensor, a blink detector, the battery status of the device, the WIFI status of the device, the Bluetooth status of the device, or querying the idle state of the device. Further improvements are contemplated by considering patient specific parameters to tailor-make the device for enhanced efficiency. For example, the shape and size of the eyelids can be obtained through state-of-the-art imaging systems like time-of-flight sensors, 3D depth imaging, etc which are generally considered for facial imaging. Using the information from the captured image, a wearable fabric or a similar device can be 3D printed to the specific personalised parameters of an individual user, further enhancing efficacy and efficiency of the management of the meibomian gland dysfunction through customised arrangement of described sensors and emitters.

This disclosure is a method and device for the treatment of meibomian gland deficiencies that uses thermal energy to increase the temperature of the eyelids and thereby the temperature of the meibomian glands causing liquification of the solidified meibum. By selecting appropriate arrangement of the carbon nanotubes or graphene layers that make up the bundle of fibres of the contemplated wearable fabric the heating effect is tailored to be efficiently absorbed by the lipids or the tissue surrounding the glands, a warming effect can be achieved without overheating other tissues. Adapting the learnings from semiconductor physics of chemical doping (i.e., addition of impurity to the semiconductor) can lead to a substantial increase in the free carrier concentration and therefore to an enhancement in the electrical and thermal conductivity.

In some embodiments since these carbon nanotubes have higher electrical conductivity or lower resistivity than for example contemporary metal wiring even at a nanoscale. In some embodiments the carbon nanofibres or graphene sheets may be combined with some impurities to marginally increase the resistivity to produce precise heating of the element. For example, boron-doped, single-walled carbon nanotubes may be used in the contemplated devices.

In another embodiment, boron in combination with nickel may be incorporated in one or more carbon electrodes. In other examples, the electrode may also incorporate one or more catalysts, for example Fe, Co, or Ni and/or their alloys. In certain embodiments, the conductive nanofibres may include at least one of carbon nanotubes, graphene, or metal wires.

In yet another preferred embodiment, the yarns in the fabric comprise at least one fibre selected from the group consisting of wool, cotton, nylon, synthetic or metals. Certain embodiments of the present disclosure relate to management of meibomian gland dysfunction via wearable fabrics that are configured with carbon nanotubes, or graphene sheets, or spider silk, or natural silk, or combinations thereof.

Certain embodiments of a composite carbon nanotube structure comprising several carbon nanotubes disposed in a matrix comprised of a metal or a metal oxide may be used in preparation of wearable fabric to treat meibomian gland dysfunction. The composite carbon nanotube structures may be used as a thermal interface device in a packaged integrated circuit device.

Certain embodiments of the disclosure include a wearable nano fabric made of composite yarn; wherein the composite yarn is prepared using a method comprising: forming a metal layer with nanowires; anodizing the metal layer to form a layer of a porous metal oxide; and forming carbon nanotubes in pores of the porous metal oxide layer.

In some embodiments, the contemplated thermal interfaces and methods include an array of carbon nanotubes aligned substantially perpendicular to the meibomian glands on the upper or lower eyelids. In other embodiments, contemplated thermal interfaces and methods include an array of carbon nanotubes aligned substantially parallel to the meibomian glands on the upper or lower eyelids; wherein the thermal interface further comprises a plurality of structures for making thermal contact with different structure within the wearable fabric; and a plurality of substantially aligned carbon nanotubes protruding substantially perpendicular from the plurality of surface structure are configured to improve thermal contact with the remaining plurality of structure to enhance the thermal efficiency of the wearable fabric to better manage the underlying meibomian gland dysfunction of the wearer.

In some other embodiments, certain frequencies of ultrasound emitters are configured such that the energy beams are focused onto the margins of the eyelids and / or parts of the eyelids such as the meibomian glands and/or the ocular adnexa. In certain embodiments, an integrated temperature sensor provides feedback to maintain the preferred temperature range. Several other modes of operation are envisaged.

In certain embodiments, the ultrasound energy may be used to aid the liquefication or emulsification of the blocked orifices, or calcification within the eyelids' margins. For example, the additional sources of ultrasound energy may be used to manage the keratinisation of the pores (openings) of the eyelid margins; wherein the ultrasound energy may be configured with resonating frequencies between 50 kHz and 1 MHz, between 1 MHz and 1.5 MHz, or between 50 kHz and 2 MHz.

In certain other embodiments, the additional sources of ultrasound energy may be used to manage the calcification of the pores (openings) of the eyelid margins; wherein the ultrasound energy may be configured with resonating frequencies of at least 50 kHz, at least 75 kHz, at least 100 kHz, at least 150 kHz, at least 300 kHz, at least 500 kHz, at least 750 kHz, at least 1 MHz, at least 1.5 MHz or at least 2 MHz.

In various exemplary embodiments presented in the current disclosure, the size, strength and the arrangement of the ultrasound energy emitters and other physical and functional characteristics of the wearable device may be contemplated.

In certain embodiments, to improve the flow of the oil through the meibomian glands, in certain embodiments, at least 1, 3, or 6 ultrasound energy emitters may be arranged in juxtaposition to the lid margins of the upper and/or lower eyelids.

In certain other embodiments, to improve the flow of meibum secretion, the number of ultrasound energy emitters may be between 1 to 3, or 2 to 4, or 2 to 5, or 1 to 6.

In certain embodiments, the number, size, and arrangement of the energy sources configured within the wearable fabric device may be such that at least 1 mm², at least 8 mm², at least 20 mm², at least 40 mm², at least 60 mm², at least 100 mm², at least 150 mm² or at least 200 mm² surface area of the eyelids is subject to the management or treatment.

In certain embodiments, the number, size and arrangement of the wearable fabric may offer the management of a surface area of the eyelids to be between 0.5 mm² and 1 mm², 1 mm² and 8 mm², 8 mm² and 20 mm², 20 mm² and 40 mm², 40 mm² and 60 mm², 60 mm² and 100 mm², 100 mm² and 150 mm², or 150 mm² and 200 mm².

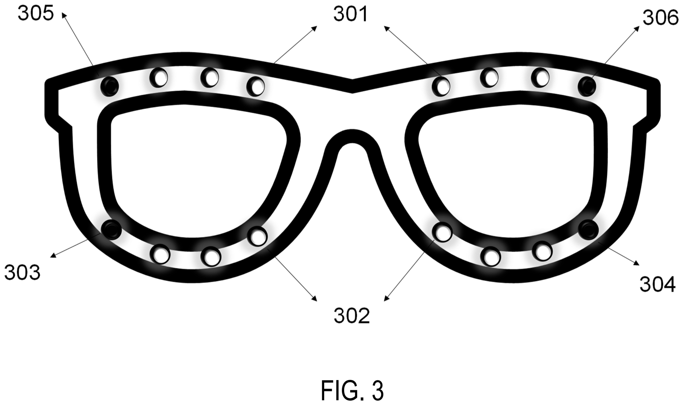

Figure 3 showcases the posterior view of the contemplated spectacle frame or wearable device which is configured with an array of sources (white-filled features) and detectors (dark-shaped features) configured to face inwards, in the direction of the upper and/or lower eyelids. The white-filled features depict the infrared light sources configured on the upper rims (301) and lower rims (302) of the contemplated spectacle or wearable frame. The dark-filled features depict the infrared temperature sensors on the lower rim (303, 304) and upper rim (305, 306) of the contemplated spectacle frame.

Various other configurations of Figure 3 may be contemplated to reduce the disclosure to practice. A device may be designed to make it simple and cheap, on the other hand, more complex embodiments with similar functionalities may be contemplated. For example, the power source for the spectacle or wearable device may either be configured within the spectacles, alternatively, the power may be sourced externally through a cable. Further, the temperature control could be via a manual switch, or alternatively an electronic chip configured onto the spectacle or wearable frame may provide feedback based on the readings obtained through the infrared sensors. Various other configurations may also be contemplated. To obtain more accurate temperature readings, it may be advantageous to disable the infrared emitters briefly before and while the temperature is measured.

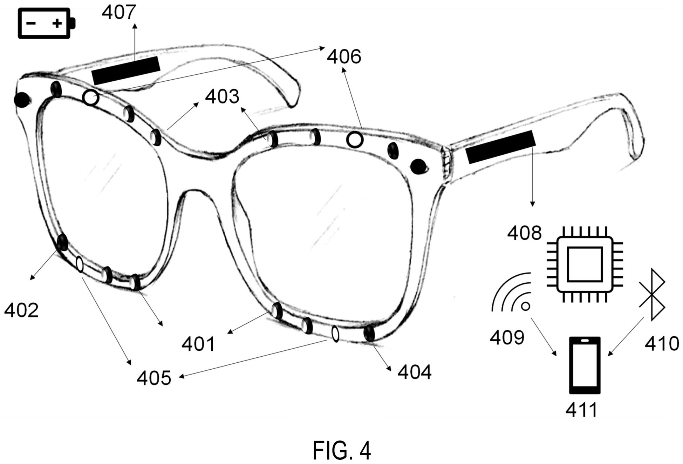

Figure 4 showcases another version of the smart spectacle frame contemplated by the current disclosure. Figure 4 illustrates a complete view of the smart spectacle frame or wearable device, which is configured with infrared sources (white-filled features) on the lower (401) and upper (403) rims of the spectacle or wearable device; and infrared sensors (402 and 404, dark-filled features) on the lower rims of the spectacle or wearable device. The spectacle or wearable device of Figure 4 is further configured with ultrasound sources on the lower (405) and upper (406) rims. To conduct the ultrasound energy to the eyelid surface, particularly focussing on the blocked or calcified orifices, an accompanying bag of liquid may be provided. For example, the bag of liquid may be biocompatible gel, bag of gel, bag of water or any other liquid that facilitates conduction and channelling the ultrasound energy to the blocked orifices. An onboard rechargeable battery (407) powers the emitters, sensors, and the microcomputer (408). The data or information processed on the micro-computer may be stored onboard, and/or transmitted to a smart device (411, phone or tablet) via use of standard Bluetooth connection (410) or wireless (409) functionality or a near-field communication chip. The transmitted data or the information may, for example be related to one of the following, the intensity setting of the infrared emitter, the power setting of the infrared emitter, the frequency setting of the ultrasound emitter, the power setting of the ultrasound emitter, the temperature reading from the infrared sensor, a blink detector, the battery status of the device, the WIFI status of the device, the Bluetooth status of the device, or querying the idle state of the device.

In certain embodiments, the size of the infrared emitting areas that may be arranged on the upper and/or lower rim of the spectacle or wearable device of the present disclosure may be approximately between 0.2 mm² and 0.4 mm², 0.3 mm² and 0.6 mm², 0.4 mm² and 0.8 mm², 0.8 mm² and 1 mm², or 0.2 mm² and 1 mm².

In certain embodiments, the size of the infrared emitting areas that may be arranged on the upper and/or lower rim of the spectacle or wearable device of the present disclosure may be at least 0.2 mm², 0.3 mm², 0.4 mm², 0.5 mm², 0.6 mm², 0.7 mm², 0.8 mm², 0.9 mm² or 1 mm².

In certain embodiments, to improve the flow of the oil through the meibomian glands, in certain embodiments, at least 3, 6, 9, 12 or 15 infrared energy sources may be arranged on the upper and/or lower rims of the spectacle or wearable device.

In certain other embodiments, to improve the flow of meibomian secretion, the number of infrared energy sources may be between 3 to 6, or 6 to 9, or 9 to 12, or 3 to 12, or 3 to 15.

In certain embodiments, the wearable spectacle frame of the current disclosure includes infrared emitters configured on the back side of the spectacle frame, facing the upper or lower eyelids, is further coupled with at least one micro-optical element to improve the delivery and efficiency of the thermal energy to the eyelids of the wearer; wherein the micro optical element is configured to produce a patch defining the area of application.

In certain other embodiments utilising wearable fabric, or smart spectacle frame or wearable frame, to improve the flow of meibomian secretion, the surface temperature of the upper or lower eyelids may be at least 40°, 41°C, 42°C, 43°C, 44°C, 45°C, or 46°C.

In certain other embodiments utilising the wearable fabric, or smart spectacle frame or wearable frame, to improve the flow of meibomian secretion, the surface temperature of the upper or lower eyelids may be between 40°C to 41°C, 41°C to 42°C, 42°C to 43°C, 43°C to 44°C, 44°C to 45°C, 41°C to 45°C, 43°C to 46°C, 40°C to 43°C, or 40°C to 46°.

In certain other embodiments utilising the wearable fabric, or smart spectacle frame or wearable frame, to improve the flow of meibomian secretion, the contemplated infrared energy sources may operate in the wavelength range of 1 µm to 1.2 µm, 1.2 µm to 1.6 µm, 1.2 µm to 1.8 µm or 1 µm to 2 µm.

In certain other embodiments utilising the wearable fabric, or smart spectacle frame or wearable frame, to improve the flow of meibomian secretion, the contemplated infrared light energy source may operate in the wavelength higher than at least 1 µm, 1.1 µm, 1.2 µm, 1.3 µm, 1.4 µm, 1.5 µm, 1.6 µm, 1.7 µm or 1.8 µm.

In certain embodiments, the number, size, and arrangement of the infrared energy sources mounted on the smart spectacle frame or wearable frame may offer the management of at least 1 mm², 4 mm², 8 mm², 12 mm², 16 mm², 20 mm², 30 mm², 40 mm², 60 mm², or 80 mm² surface area of the eyelids.

In certain embodiments, the number, size and arrangement of the infrared energy sources mounted on the spectacle or wearable frame may offer the management of a surface area of the lids between 1 mm² and 8 mm², 8 mm² and 20 mm², 20 mm² and 40 mm², 30 mm² and 60 mm², 40 mm² and 70 mm², 50 mm² and 80 mm², or 20 mm² and 80 mm².

In some embodiments of the present disclosure, the contemplated spectacle or wearable frame may be fitted with multiple rows of infrared sources that illuminate the eyelids and lid margins either continuously or intermittently at pre-programmed or user-triggered intervals.

In one implementation of certain embodiment, it may be desirable or preferable to integrate a sensor that may detect closed eyelids, which can modulate the heating energy applied based on the open or closed state of the eyelids.

For example, the strength and duration of the pulse of the infrared source may be automatically adjusted depending on whether the eye is open or closed. For example, in a closed eye condition, the strength and duration of the pulse can be programmed to be substantially greater than the strength and duration contemplated in an open eye condition. Such a programming function may provide the user full control over their treatment and may also increases efficiency as more eyelid tissue is exposed to the treatment while the lids are closed than when they are open otherwise.

In yet another exemplary embodiment, another variation may be contemplated for application overnight on the closed eyes; wherein ultrasonic energy may be used to manage the cavitation of blocked orifices. By using an array of individual infrared LED, a pattern of selectively illuminated areas can be created. This could be advantageous for stimulating the increase in lipid generation and flow.

Most effective treatment effects can be obtained by using infrared light at wavelengths between 1.2 or 1.7 µm. These are the regions where energy is absorbed by the lipids most efficiently.

In some other contemplate variations of the present disclosure, not every user may be a spectacle wearer, in such instances, the contemplated device of the current disclosure may be a wearable frame without lenses, or with plano lenses or with sunglasses, as disclosed herein.

The information, with respect to functional and structural aspects of the disclosure disclosed herein, should be construed merely as a representative basis for teaching a person skilled in the art to employ the disclosure and variations of those embodiments. The present disclosure is not restricted to any particular construction described and illustrated here but should be constructed to cohere with all modifications that may fall within the scope of the appended claims. Although shown and described are what is predicted to be practical and preferred embodiments, it is apparent that departures from specific designs and/or methods described will suggest themselves to those skilled in the art and may be used without departing from the spirit and scope of the disclosure. The disclosure described with reference to one or more embodiments, some of which are illustrated by accompanying figures. The embodiments or figures are provided by way of explanation and are not to be construed as limiting to the scope of the disclosure.

Certain embodiments of the present disclosure relate to management of meibomian gland dysfunction via spectacle frame or wearable based devices that are configured such that a certain wavelength of the infrared sources, and additionally certain frequencies of ultrasound energy, are configured such that the energy beams are focused onto the eyelids and / or parts of the eyelids such as the meibomian glands and/or the ocular adnexa. In yet other embodiment of the present disclosure, other forms of energy, for example, energy in a continuous form or pulsed form having a relatively high peak energy may also be useful in the meibomian gland treatments. In various exemplary embodiments presented in the current disclosure, the size, strength and the arrangement of the energy sources and other physical and functional characteristics of the spectacle or wearable device may be contemplated.

Further, in addition to the discomfort driven by the meibomian gland dysfunction, the clogged meibomian glands could lead to other resulting eye infections, including stye, chalazions, or blepharitis. Several factors are involved in diagnosing and treating dry eyes, including tear production, quality of the tear film, lid hygiene, natural bacteria and even diet. This disclosure also contemplates a multifaceted approach to manage meibomian gland deficiencies which is envisaged through a personalised kit, as disclosed herein. Accordingly, the present disclosure offers convenient combination and personalised therapy for improving overall eyelid hygiene and ameliorate meibomian gland deficiencies.

The advent of current day technology has offered the possibility of miniaturisation of complex optical, electrical, electronic, or mechanical systems to portable, handheld, relatively economical, devices that can perform the same complex tasks of the previous decade. The present disclosure of a kit for managing meibomian gland dysfunction includes a diagnostic non-invasive pocket imaging system for a user. Other advantages of the current disclosure, such as, replacing the need for relatively large, expensive, cumbersome and energy inefficient current systems with newer alternatives that are user-friendly, energy efficient, precise heating elements, relatively inexpensive and that may eliminate the need for a substantial aid from the medical practitioner to conduct the procedure and manage the meibomian gland dysfunction and other dry eye conditions will become evident.

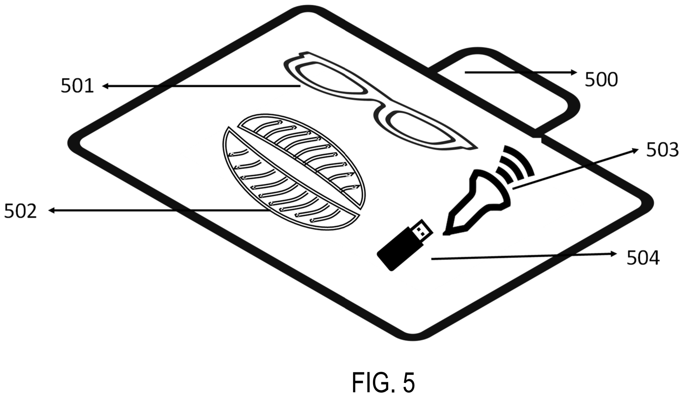

Figure 5 showcases a kit to manage meibomian gland deficiencies of a wearer, the kit comprising: a wearable frame, the wearable frame configured with at least one emitter and one sensor; a wearable fabric, the wearable fabric comprising of a bundle of composite yarn of electromechanical materials; and a handheld portable ultrasound device, the ultrasound device comprising of at least one ultrasound emitter and one sensor to image the meibomian glands of the wearer. The kit (500) for managing meibomian gland deficiencies, includes a spectacle frame (501), a wearable fabric (502), a handheld ultrasound device (503), an USB data transmitter (504), as disclosed herein. The functioning of the spectacle frame and wearable fabric are disclosed in Figures 1 to 4.

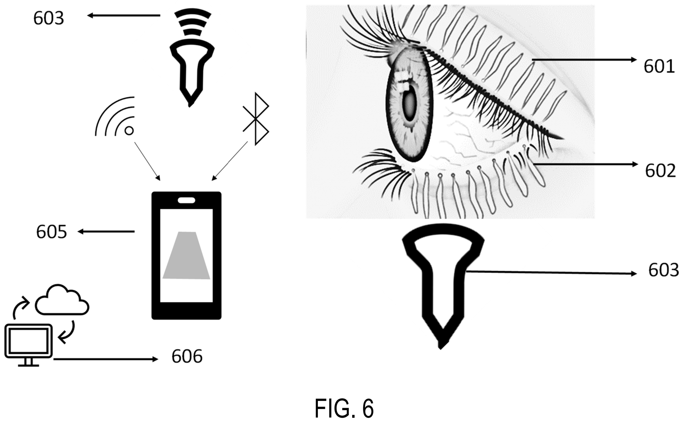

Figure 6 showcases the functioning of a handheld ultrasound imaging system (603) on the upper (601) and lower (602) eyelids of a wearer, which relays the measured signals onto a handheld device (605, for example a mobile phone or a tablet) via either a Bluetooth module or a WIFI module. The data collected from the user may be sent and stored on the cloud (606).

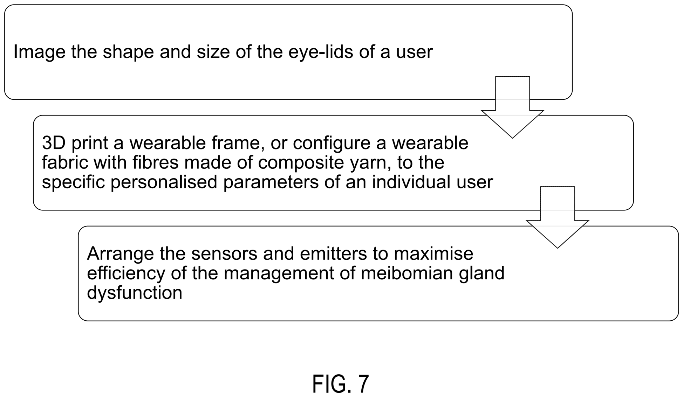

Figure 7 illustrates a flow chart of a method that may be used to manage an eye according to certain aspects of the present disclosure, as disclosed herein. In certain embodiments of the kit, the shape, size, and other features of the eyelids of a user may be imaged using time-of-flight sensors or 3D depth cameras to allow for 3D reconstruction of the eyelid and facial contour. Further, the imaged information about the eyelids and facial contours may be used as an input into a traditional or dedicated 3D printer to obtain a spectacle or wearable frame, which may be further configured to include contemplated infrared sensors and emitters to manage the meibomian gland deficiencies, as disclosed herein. In yet another embodiment of the kit, the imaged information about the eyelids and facial contours may be used as an input into a traditional or dedicated 3D printer to obtain a wearable mask.

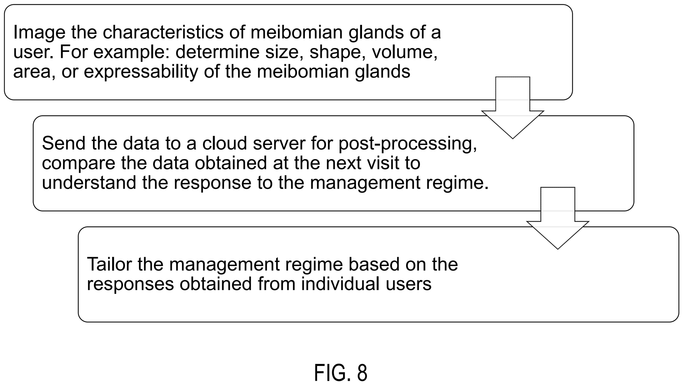

Figure 8 provides a further improvement of the contemplated disclosure, wherein parameters of the individual user can be used to tailor-make the device for enhancing efficiency. Figure 8 further illustrates a flow chart of a method directed by personalised data of the user that may be used to manage an eye according to certain aspects of the present disclosure, as disclosed herein. In one embodiment of the kit disclosed herein, the data describing the characteristics of meibomian glands, for example, shape, size, area, volume of the meibomian glands or expressability (i.e., the ability of the meibomian gland to release meibum upon application of pressure) can be collected as a function of time.

In certain embodiments of the kit, the collected information outlining the characteristics of the meibomian gland may be displayed on a handheld device, for example tablet or phone. In one embodiment, a substantially non-invasive method and/or device is selected from the kit, for example, a spectacle or wearable frame, which uses, at least in part, infrared energy to warm up the eyelids, and thereby increasing the temperature of the meibomian glands through principles of thermal conduction. For example, a substantially non-invasive method and/or device is selected from the kit. For example, a wearable fabric, which uses, at least in part, thermal energy to warm up the eyelids, and thereby increasing the temperature of the meibomian glands through principles of thermal conduction, which also may be referred to as Joule heating.

In certain embodiments of the disclosure, the contemplated wearable fabric of the kit, is further configured by selecting a specific arrangement of the fibres that offer thermal radiation that is most efficiently absorbed by the lipids or the tissue surrounding the glands, wherein a warming effect can be achieved without substantially overheating other tissues around the ocular adnexa. In certain embodiments of the kit, an integrated, non-contact, temperature sensor may be configured onto to the spectacle frame. Several other modes of operation may be envisaged by a person skilled in the art.

In certain embodiments of the kit, an integrated, temperature sensor may be weaved into the wearable fabric providing feedback to maintain the preferred temperature range of heat application. In certain embodiments of the kit, the wearable fabric may also be configured with additional rows of miniaturised ultrasound emitters that may be positioned closed to the upper and/or lower eyelid margins to either function in a continuous or intermit mode or at a pre-programmed mode or via user-triggered intervals for liquefaction of the calcified or keratinised orifices of the eyelids of the wearer.

In another implementation of the present disclosure of the kit, the wearable fabric may be configured such that the user may be able to keep their eyes open for any routine visual tasks that they would otherwise take part on a normal day; while in another implementation of the present disclosure, the wearable fabric may be configured such that the user may only be able to use the wearable device in close eye conditions. In embodiments of the kits designed specifically for closed eye application, it may be preferable to integrate a sensor that detects closed eyelids, and heating energy is only applied while the eyelids are closed for prolonged periods. This may provide the user full control over the treatment and also increases efficiency as more eyelid tissue is exposed to the treatment while the lids are closed.

Other variations to apply the treatment overnight on the closed eyes may be contemplated by a person skilled in the art.

In certain embodiments of the present disclosure of the kit, a fabric configured with a matrix of electro-mechanical materials or fibres that are substantially made out of carbon nanotubes, or graphene plates or sheets or spider silk fibres or natural silk fibres or other metal fibres, and the bundles of such fibres are further configured to be substantially aligned along at least one specified direction or axis of the eyelids of the wearer or user of the device.

In some embodiments of the kit, the apparatus may comprise of multiple layers of nanofibres substantially made of carbon nanotubes and/or graphene plates or sheets which may be further configured to suit the shape of the eyelids of an individual wearer. A plurality of electro-mechanical configurations of the nanofibres at plurality of locations on the eyelids are contemplated in the current disclosure of a kit. The alignment of the nanofibres of the contemplated wearable device may further facilitate heating and mechanical vibrations in at least one specific direction or axis of the eyelids of the wearer or user of the device.

Various other configurations of the kit can be contemplated to reduce the disclosure to practice. A device of the kit may be designed to make it simple and cheap, on the other hand, more complex embodiments may be contemplated. For example, the power source may be configured as an extension of the wearable fabric of the kit, on the other hand, the power may be sourced externally through a cable. The wearable fabric of the kit may be further configured with ultrasound emitters close to the lower and upper margins of the eyelids. An onboard rechargeable battery sources the emitters, sensors, and the micro-chip computer may be contemplated. The data or information processed on the micro-computer may be transmitted to a smart device via use of standard Bluetooth connection or wireless functionality.

### EXAMPLE CLAIM SET A - WEARABLE FABRIC

A wearable fabric device configured in juxtaposition with the eyelids of a wearer for managing meibomian gland dysfunction of the wearer;
wherein the wearable fabric device comprises of substantially stretchable conductive composite yarn materials including one or more of the following: a carbon nano tube, a graphene sheet, natural, spider or artificial silk, a metal or an alloy, cotton or denim, and an elastic formed of a polymer or copolymer of a polyamide, a polyester, a polyolefin, and mixtures thereof;
wherein the wearable fabric device further comprises one or more of the following components: an infrared emitter, an infrared sensor, an ultrasound emitter, a micromotor, a microcomputer, a Bluetooth module, a near-field communication chip, a Wi-Fi module, and a rechargeable battery power source;
wherein the wearable fabric device in use applies thermal energy externally onto the upper and/or lower eyelids of the wearer; such that the application of the thermal energy causes liquification of solidified oil (meibum) of the meibomian glands of the wearer;
wherein the wearable fabric device in use applies mechanical energy externally onto the upper or lower eyelids of the wearer; such that the application of the mechanical energy generates a regulated directional force to express the obstruction located within the meibomian glands through its orifices;
wherein the wearable fabric device in use applies ultrasound energy focussing within the proximity of the orifices of the upper or lower eyelids of the wearer; such that the application of the ultrasound energy facilitates emulsification of the hardened meibum at the orifices of the upper or lower eyelids of the wearer; and
wherein the wearable fabric device in use provides a closed-loop feedback and an active control mechanism to finetune the thermal and mechanical energy applied onto the eyelids of the wearer using a mobile application.

The wearable fabric device of one or more preceding examples of set A, wherein the fabric is configured to take into consideration the wearer specific information of the eyelids, face, or ocular orbit.

The wearable fabric device of one or more preceding examples of set A, wherein the number of infrared emitters configured in juxtaposition with the lid margins is between 2 and 6, between 2 and 8, between 3 and 9, or between 4 and 10.

The wearable fabric device of one or more preceding examples of set A, wherein the at least one infrared emitter has a wavelength of at least 950 nm, at least 1050 nm, at least 1150 nm, at least 1300 nm, at least 1500 nm, or at least 1700 nm.

The wearable fabric device of one or more preceding examples of set A, wherein the area of application of thermal energy is at least 3 mm² on the upper or lower eyelids of the wearer.

The wearable fabric device of one or more preceding examples of set A, wherein the area of application of thermal energy on the upper or lower eyelids of the wearer is between 3 and 9 mm², 3 and 12 mm², or 3 and 18 mm².

The wearable fabric device of one or more preceding examples of set A, wherein the surface temperature on the upper or lower eyelid is at least 40 °C, at least 40.5 °C, at least 41 °C, at least 41.5 °C, at least 42 °C, at least 42.5 °C, at least 43 °C, or at least 43.5 °C.

The wearable fabric device of one or more preceding examples of set A, wherein the surface temperature on the upper or lower eyelid is between 40 °C and 43.5 °C, between 41 °C and 42.5 °C, between 41.5 °C and 43 °C, or between 41.5 °C and 44.5 °C, or between 41.5 °C and 46.5 °C.

The wearable fabric device of one or more preceding examples of set A, wherein the thermal energy is applied continuously and uniformly across the upper or lower eyelids for a period of 10 to 60 minutes.

The wearable fabric device of one or more preceding examples of set A, wherein the thermal energy is applied discontinuously and in a pulsating mode across the upper or lower eyelids for a period of 10 to 60 minutes such that the amplitude and frequency of the pulse mode of operation provides a location gradient in eyelid temperature.

The wearable fabric device of one or more preceding examples of set A, wherein the applied thermal energy has a frequency between 10 and 30 Hz for continuous heating.

The wearable fabric device of one or more preceding examples of set A, wherein the applied thermal energy has a frequency between 0.5 and 5 Hz for pulse heating.

The wearable fabric device of one or more preceding examples of set A, wherein the thermal energy is applied across the upper or lower eyelid in a gradient manner, such that the application of thermal energy to the orifices of the meibomian glands is at least 2°C greater than the thermal energy applied to the top of the meibomian glands , such that the applied gradient in temperature facilitates a smooth flow of the liquefied meibum from the glands towards the orifices of the eyelids of the wearer.

The wearable fabric device of one or more preceding examples of set A, wherein the at least one micromotor is configured to provide a vibration in the tangential direction of the eyelids of the wearer.

The wearable fabric device of one or more preceding examples of set A, wherein the vibrations of the at least one micromotor to generate a pressure wave from the origin to the orifice of the meibomian glands with a pressure range of 0.005 to 0.5 N/mm² and a frequency range from 0.5 to 3 Hz.

The wearable fabric device of one or more preceding examples of set A, wherein the at least one micromotor is configured to provide a rotatory distribution forces in the tangential direction of the eyelids of the wearer.

The wearable fabric device of one or more preceding examples of set A, wherein the at least one ultrasound emitters is configured in juxtaposition with the upper lid margin and at least one configured in juxtaposition with the lower eyelid margin.

The wearable fabric device of one or more preceding examples of set A, wherein the number of ultrasound emitters configured in juxtaposition with the lid margins is between 2 and 6, between 2 and 8, or between 3 and 9.

The wearable fabric device of one or more preceding examples of set A, wherein the at least one ultrasound emitter has a frequency that is at least 100 kHz, at least 200 kHz, at least 300 kHz, at least 400 kHz or at least 500 kHz.

The wearable fabric device of one or more preceding examples of set A, wherein the at least one ultrasound emitter has a frequency range that is 100 kHz and 500 kHz, 200 kHz and 500 kHz, or 100 kHz and 300 kHz.

The wearable fabric device of one or more preceding examples of set A, wherein the fabric is held in place against the upper or lower eye lids of the using an elastic headband; wherein the elastic headband houses a rechargeable battery to power up the device at the back of the head.

A method for managing meibomian gland dysfunction of the wearer, the method comprising an executable mobile application, the application comprising: (a) a wearable apparatus communication module arranged to communicate with a wearable device in accordance with claim examples of Set A; (b) a data processing module arranged to process data received by the wearable device; and (c) a user interface module arranged to provide a user interface for gathering user specific data for storage in the user database and to prompt, to the user, recommendations for modifying the management or better treatment user's meibomian gland dysfunction.

A method for managing meibomian gland dysfunction of the wearer, the method utilising the wearer or user specific data comprises a plurality of thresholds that may be set or modified by the wearer or user via the user interface module of the mobile application.

The wearable fabric device of one or more preceding claim examples of set A, wherein the executable mobile application is used to alter the properties of the application of thermal or mechanical energy to the upper or lower eyelids of the wearer.

A method for managing meibomian gland dysfunction of the wearer, the method comprising the steps of: (a) receiving data from a wearable apparatus in accordance with any one of claim examples of set A; (b) processing the received data in a data processing unit via the mobile application; and (c) comparing at least a set of the processed data with at least one criterion related to normative data on meibomian gland dysfunction or against the baseline measurement of the wearer; and (d) generating or modifying the thermal or mechanical energy as part of personalising the treatment using the wearable device.

### EXAMPLE CLAIM SET B - A SPECTACLE BASED DEVICE FOR MGD

A spectacle frame for managing meibomian gland dysfunction of a wearer;
wherein the spectacle frame comprises one or more of the following components: an infrared emitter, an infrared sensor, an ultrasound transducer, a microcomputer, a Bluetooth module, a near-field communication chip, a Wi-Fi module, and a rechargeable battery power source;
wherein the spectacle frame directs thermal energy onto the upper and/or lower eyelid surfaces of the wearer; such that the application of the thermal energy causes liquification of at least some of the solidified oil (meibum) of the meibomian glands of the wearer in both open and closed eye conditions;
wherein the spectacle frame in use provides a closed-loop feedback and an active control mechanism to finetune the thermal energy applied onto the eyelid surface of the wearer using a mobile application.

The spectacle frame of one or more preceding examples of set B, wherein the shape of the spectacle frame is configured to take into consideration the wearer specific information of the eyelids, face, or ocular orbit.

The spectacle frame of one or more preceding examples of set B, wherein the number of infrared emitters configured on the back side of the spectacle frame is between 2 and 8, between 4 and 12, or between 2 and 16; wherein the arrangement on the backside of the spectacle frame is such that, they direct thermal energy towards the lids of the wearer.

The spectacle frame of one or more preceding examples of set B, wherein each of the infrared emitters configured on the back side of the spectacle frame is further coupled with at least one micro-optical element to improve the delivery and efficiency of the thermal energy to the eyelids of the wearer; wherein the micro optical element is configured to produce a patch defining the area of application.

The spectacle frame of one or more preceding examples of set B, wherein the infrared emitter has a wavelength of at least 950 nm, at least 1050 nm, at least 1150 nm, at least 1300 nm, at least 1500 nm, or at least 1700 nm.

The spectacle frame of one or more preceding examples of set B, wherein the area of application of thermal energy is at least 3 mm² on the upper or lower eye lids of the wearer.

The spectacle frame of one or more preceding examples of set B, wherein the area of application of thermal energy on the upper or lower eye lids of the wearer is between 3 and 6 mm², 3 and 12 mm², or 3 and 18 mm².

The spectacle frame of one or more preceding examples of set B, wherein the surface temperature on the upper or lower eyelid is at least 40 °C, at least 40.5 °C, at least 41 °C, at least 41.5 °C, at least 42 °C, at least 42.5 °C, at least 43 °C, or at least 43.5 °C.

The spectacle frame of one or more preceding examples of set B, wherein the surface temperature on the upper or lower eyelid is between 40 °C and 43.5 °C, between 41°C and 42.5 °C, between 41.5 °C and 43 °C, or between 41.5 °C and 44.5 °C, or between 41.5 °C and 46.5 °C.

The spectacle frame of one or more preceding examples of set B, wherein the number of infrared sources is at least 3 in the upper rim of the spectacle and at least 3 in the lower rim of the spectacle.

The spectacle frame of one or more preceding examples of set B, wherein the number of ultrasound emitters configured in juxtaposition with the lid margins is between 2 and 6, between 2 and 8, or between 3 and 9.

The spectacle frame of one or more preceding examples of set B, wherein each of the ultrasound emitter has a frequency that is at least 100 kHz, at least 200 kHz, at least 300 kHz, at least 400 kHz or at least 500 kHz.

The spectacle frame of one or more preceding examples of set B, wherein each of the one ultrasound emitter has a frequency range that is 100 kHz and 500 kHz, 200 kHz and 500 kHz, or 100 kHz and 300 kHz.

The spectacle frame of one or more preceding examples of set B, wherein each of the ultrasound emitter is used in conjunction with a silicone bag filled with fluid that abuts the spectacle frame ultrasound source and the close eyelids to facilitate the transfer of ultrasound energy to the targeted locations on the eyelid.

The spectacle frame of one or more preceding examples of set B, wherein the thermal energy is applied continuously and uniformly across the upper or lower eyelids for a period of 10 to 60 minutes.

The spectacle frame of one or more preceding examples of set B, wherein the thermal energy is applied discontinuously and in a pulsating mode across the upper or lower eyelids for a period of 10 to 60 minutes such that the amplitude and frequency of the pulse mode of operation provides a location gradient in eyelid temperature; wherein the applied thermal energy pulse has a frequency between 0.1 and 30 Hz.

A method for managing meibomian gland dysfunction of the wearer, the method comprising an executable mobile application, the application comprising: (a) a wearable apparatus communication module arranged to communicate with a spectacle in accordance with the claim examples of Set B; (b) a data processing module arranged to process data received by the spectacle; and (c) a user interface module arranged to provide a user interface for gathering user specific data for storage in the user database and to prompt, to the user, recommendations for modifying the management or better treatment user's meibomian gland dysfunction.

A method for managing meibomian gland dysfunction of the wearer, the method utilising the wearer or user specific data comprises a plurality of thresholds that may be set or modified by the wearer or user via the user interface module of the mobile application.

The wearable spectacle frame of one or more preceding examples of set B, wherein the executable mobile application is used to alter the properties of the application of thermal energy to the upper or lower eyelids of the wearer.

A method for managing meibomian gland dysfunction of the wearer, the method comprising the steps of: (a) receiving data from the spectacle in accordance with any one of the claim examples of Set B; (b) processing the received data in a data processing unit via the mobile application; and (c) comparing at least a set of the processed data with at least one criterion related to normative data on meibomian gland dysfunction or against the baseline measurement of the wearer; and (d) generating or modifying the thermal or mechanical energy as part of personalising the treatment using the wearable device.

### EXAMPLE CLAIM SET C - A KIT TO TREAT MEIBOMIAN GLAND DYSFUNCTION

A kit to manage meibomian gland deficiencies of a wearer, the kit comprising: a wearable fabric, a spectacle frame, a handheld portable ultrasound imaging device and a mobile application; wherein the wearable fabric or spectacle frame is configured taking into consideration at least one user specific measurement;
wherein the wearable fabric of the kit comprises of substantially stretchable conductive composite yarn materials including one or more of the following: carbon nano tubes, graphene sheets, natural silk, spider silk, artificial silk, pure metal, metal alloy, cotton, denim, or elastic formed of a polymer or copolymer of a polyamide, a polyester, a polyolefin, and mixtures thereof;
wherein the wearable fabric of the kit further comprises of one or more of the following components: a plurality of infrared emitters, a plurality of infrared temperature, a plurality of ultrasound transducers, a plurality of micromotors, a microcomputer, a Bluetooth module, a near-field communication chip, a Wi-Fi module, and a rechargeable battery power source;
wherein the plurality of infrared emitters of the wearable fabric in use provides a desired level of thermal energy to be applied externally onto the eyelids of the wearer to cause liquification of solidified oil (meibum) within the meibomian glands of the wearer in closed eye condition;
wherein the plurality of micromotors of the wearable fabric in use provides a desired level of mechanical energy or vibrational force to be applied externally onto the eyelids of the wearer to generate a regulated directional force to express solidified meibum through orifices of the meibomian gland of the wearer;
wherein the plurality of ultrasound transducers of the wearable fabric in use applies ultrasound energy purposefully focussing or directed at orifices of the eyelids of the wearer to facilitate emulsification of the solidified meibum at eyelid orifices of the wearer;
wherein the wearable fabric of the kit in use provides a closed-loop feedback to the wearer and an active control mechanism to finetune the thermal energy and mechanical forces applied onto the eyelids of the wearer using a mobile application;
wherein the spectacle frame of the kit comprises one or more of the following components: a plurality of infrared emitters, a plurality of infrared sensors, a plurality of ultrasound transducers, a microcomputer, a Bluetooth module, a near-field communication chip, a Wi-Fi module, and a rechargeable battery power source;
wherein the plurality of infrared emitters of the spectacle frame focuses thermal energy onto the eyelid surfaces of the wearer to cause liquification of solidified oil (meibum) of the meibomian glands of the wearer in open eye condition;
wherein the spectacle frame of the kit in use provides a closed-loop feedback and an active control mechanism to finetune the thermal energy applied onto the eyelid surface of the wearer using a mobile application;
wherein the handled portable ultrasound imaging device comprising an ultrasound transducer probe and an ultrasound receiver for monitoring the health of the meibomian glands of the wearer utilises multi-frequency self-focussing ultrasound imaging principles.

The kit of one or more the preceding claims of set C, wherein the plurality of infrared emitters configured within the wearable fabric or spectacle frame are at least 3, 6, 9, or 12 emitters and the plurality of sensors configured within the wearable fabric are at least 2, 3, or 4.

The kit of one or more the preceding claims of set C, wherein the plurality of infrared emitters configured within the wearable fabric or spectacle frame has the wavelength of at least 1 µm, 1.1 µm, 1.2 µm, 1.3 µm, 1.4 µm, 1.5 µm, or 1.6 µm.

The kit of one or more the preceding claims of set C, wherein the at least one ultrasound transducer on the wearable frame or spectacle frame has the frequency that is at least 100 kHz, at least 200 kHz, at least 300 kHz, at least 400 kHz or at least 500 kHz.

The kit of one or more the preceding claims of set C, wherein the at least one ultrasound emitter on the wearable frame or spectacle frame has the frequency that is 100 kHz and 500 kHz, 200 kHz and 500 kHz, or 100 kHz and 300 kHz.

The kit of one or more the preceding claims of set C, wherein the plurality of ultrasound transducers of the wearable fabric are purposefully configured in juxtaposition with the upper or lower lid margin.

The kit of one or more the preceding claims of set C, wherein the fabric is held in place against the upper or lower eyelids of the using an elastic headband; wherein the elastic headband houses a rechargeable battery to power up the device at the back of the head.

The kit of one or more the preceding claims of set C, wherein the handheld portable ultrasound device is wireless.

The kit of one or more the preceding claims of set C, wherein the handheld portable ultrasound device is used to provide feedback on the ongoing management of the meibomian gland deficiencies of the wearer.

The kit of one or more the preceding claims of set C, wherein the handheld portable ultrasound device is used to provide feedback on the ongoing management of the meibomian gland deficiencies of the wearer.

The kit of one or more the preceding claims of set C, wherein the thermal energy from the wearable fabric is applied continuously and uniformly across the upper or lower eyelids for a period of 10 to 60 minutes.

The kit of one or more the preceding claims of set C, wherein the thermal energy is applied discontinuously and in a pulsating mode across the upper or lower eyelids for a period of 10 to 60 minutes such that the amplitude and frequency of the pulse mode of operation provides a location gradient in eyelid temperature; wherein the applied thermal energy pulse has a frequency between 0.1 and 30 Hz.

The kit of one or more the preceding claims of set C, wherein the thermal energy is applied across the upper or lower eyelid in a gradient manner, such that the application of thermal energy to the top of the meibomian glands is at least 2°C greater than the thermal energy applied to the orifices of the meibomian glands, such that the applied gradient in temperature facilitates a smooth flow of the liquefied meibum from the glands towards the orifices of the eyelids of the wearer.

The kit of one or more the preceding claims of set C, wherein the at least one micromotor is configured to provide a vibration in the tangential direction of the eyelids of the wearer; wherein the vibrations of the at least one micromotor to generate a pressure wave from the origin to the orifice of the meibomian glands with a pressure range of 0.005 to 0.5 N/mm² and a frequency range from 0.5 to 3 Hz.

The kit of one or more the preceding claims of set C, wherein the at least one micromotor is configured to provide a rotatory distribution forces in the tangential direction of the eyelids of the wearer.

The kit of one or more the preceding claims of set C, wherein the feedback for the ongoing management of the meibomian gland deficiencies of the user may include data on the following characteristics of the meibomian glands: size, shape, texture, area, volume, or expressability of the glands.

## Claims

1. A wearable device for managing meibomian gland dysfunction of a wearer, the device configured to be worn in juxtaposition with the eyelids of the wearer, the device comprising:
a pair of material portions (105a, 105b, 106a, 106b, 201, 202) for left and right eyes of the wearer, each material portion configured to contact an upper and lower portion of eyelids of the left and right eyes of the wearer;
wherein each material portion comprises one or more of: carbon nanotube, graphene plate or sheet, natural, spider or artificial silk, cotton, denim, a metal, metal nanowires or an alloy, or an elastic formed of a polymer or copolymer of a polyamide, a polyester, a polyolefin, and mixtures thereof;
at least one thermal energy delivery assembly (109a, 109b) configured to deliver thermal energy within each material portion to provide heating to the contacted eyelid at a temperature sufficient to liquefy meibum and facilitate meibomian gland expression;
at least one vibration arrangement (109c) within each material portion configured to deliver mechanical energy to impart massaging motion onto the eyelid to further facilitate meibum outflow, the vibration arrangement comprising at least one micromotor or piezo-activator;
a closed-loop feedback arrangement or an active control mechanism that is configured to finetune applied thermal energy exerted by the device on the wearer's eyelid;
a microcomputer (205) in communication with the thermal energy delivery assembly and the vibration arrangement, the microcomputer with a data processing module being programmed to:
receive user data including target temperature and vibration settings; and
finetune operation of the thermal energy delivery assembly to maintain a desired eyelid temperature range;
wherein the device is configured to be operated repeatedly by a wearer at home without eye care professional assistance; and
wherein the device is configured to take into consideration wearer-specific information of the eyelids, face, or ocular orbit.

2. The wearable device of claim 1, wherein the at least one micromotor or piezo-activator is configured to provide a vibration to the eyelids of the wearer; wherein the vibration generates a pressure wave from the origin to the orifice (104) of the meibomian glands (102, 103) with a pressure range of 0.005 to 0.5 N/mm².

3. The wearable device of claim 2, wherein the vibration is in the tangential direction of the eyelids.

4. The wearable device of claims 1 to 3, wherein the alignment of the material portions of the wearable device facilitates delivery of mechanical energy in at least one specific direction or axis of the eyelids of the wearer of the device.

5. The wearable device of claims 1 to 4, wherein the thermal energy is applied for a period of 10 to 60 minutes to the upper or lower eyelids.

6. The wearable device of claims 1 to 5, wherein the thermal energy is applied continuously and uniformly across the upper or lower eyelids.

7. The wearable device of claims 1 to 5, wherein the thermal energy is applied discontinuously and in a pulsating mode across the upper or lower eyelids such that the amplitude and frequency of the pulse mode of operation provides a location gradient in eyelid temperature.

8. The wearable device of claims 1 to 5, wherein the thermal energy is applied continuously or for pulse heating with a frequency between 0.5 and 5 Hz.

9. The wearable device of claims 1 to 5, wherein the thermal energy is applied discontinuously and in a pulsating mode across the upper or lower eyelids; wherein the applied thermal energy pulse has an amplitude of at least 2°C and wherein the applied thermal energy pulse has a frequency between 0.5 and 5 Hz.

10. The wearable device of claim 9, wherein the thermal energy is applied across the upper or lower eyelid in a gradient manner, such that the applied gradient in temperature facilitates a smoother flow, stimulates or improves flow, of the liquefied oil (meibum) from the glands towards the orifices (104) of the eyelids of the wearer.

11. The wearable device of claims 1 to 10, wherein the surface temperature on the upper or lower eyelid is maintained between 40°C and 46.5°C when the device is in use.

12. The wearable device of claims 1 to 11, wherein the surface temperature on the upper or lower eyelid is at least 40°C.

13. The wearable device of claims 1 to 12, wherein the area of application of thermal energy on the upper and lower eyelids of the wearer is at least 20 square mm.

14. The wearable device of claims 1 to 13, wherein the number, size, and arrangement of the energy sources configured within the wearable device is such that at least 20 mm² surface area of the eyelids is subject to the management or treatment.

15. The wearable device of claims 1 to 14, wherein the microcomputer (205) gathers user-specific data providing prompts to the wearer with suggested recommendations.

16. The wearable device of claims 1 to 15, wherein the combination of controlled heating, vibration, and closed-loop feedback (or active control mechanism) is operable to promote expression of meibomian glands (102, 103) in a substantially non-invasive manner.

17. The wearable device of claims 1 to 16, wherein a plurality of micromotors provide a desired level of mechanical energy or vibrational force or a regulated directional force externally onto the eyelids of the wearer to express the obstruction located within the meibomian glands (102, 103) through its orifices (104).
